# EUROPEAN PATENT SPECIFICATION

(11) **EP 2 919 834 B1**
(45) Date of publication and mention of the grant of the patent: **16.05.2018**
(21) Application number: 13795200.8
(22) Date of filing: 15.11.2013
(51) Int. Cl.: A61M 5/24, A61M 5/32

(54) **INJECTION NEEDLE ASSEMBLY**
INJEKTIONSNADELEINHEIT
ENSEMBLE AIGUILLE D'INJECTION

(30) Priority: 19.11.2012 SE 1251303; 19.11.2012 US 201261727765 P
(43) Date of publication of application: 23.09.2015
(73) Proprietor: Carebay Europe Ltd., Sliema, SLM 1643 (MT)
(72) Inventor: KARLSSON, Sebastian, 611 72 Stigtomta (SE); DANIEL, Mattias, 18734 Täby (SE)
(86) International application number: PCT/EP2013/073915
(87) International publication number: WO 2014/076225

(56) References cited:
- WO-A1-2009/150078
- WO-A1-2010/094916
- WO-A1-2011/112136
- WO-A1-2011/126439
- WO-A2-2011/039230

## Description

### TECHNICAL AREA

The present invention relates to an injection needle assembly and in particular an injection needle assembly comprising a retainer member attachable to a medicament container and a cap member removably attached to the retainer member.

### BACKGROUND OF INVENTION

Different types of injection needle assemblies have been developed during a number of years, where the aim is to have a more or less complete assembly that is attached to a medicament container. Regarding the aspect of attachment of injection needles to medicament containers, there is often a requirement that the rear end of the injection needle should protrude into the interior of the container.

This could be a drawback if the medicament reacts with the material of the needle when exposed for a period of time. In that respect it would be desirable to have the rear part of the needle outside the container until the injection is to be performed. On the other hand, this then requires that the rear end of the needle could be moved fairly easily into the container and also that the rear end of the needle is kept in a sterile environment until it is moved into the container.

A novel solution to these requirements is presented in the document WO 2009/150078. In '078, an injection needle assembly is disclosed provided with a retainer member attachable to a medicament container. The retainer member is arranged with a central passage, in which a hub is arranged. The hub holds an injection needle having a proximal and distal pointed end. Further a cap member is removably attached to the retainer member. In an initial position, both ends of the injection needle are protected by the assembly, keeping the injection needle sterile.

The assembly is designed such that when the cap member is removed in order to deliver a dose of medicament, the cap member holder is moved in a distal direction in relation to the retainer member such that the distal pointed end of the injection needle penetrates a membrane of the medicament container in a medicament delivery device to which the injection needle assembly is attached. Document WO2011/126439 describes an analogous injection needle assembly and mode of operation. These assemblies work very well in most instances and the injection needle is kept sterile. However, in some rare instances, there might be a risk that foreign matter may enter into the interior of the assembly through the interface between the cap member and the retainer member, in particular if there is some relative movement between the two parts. Therefore it would be advantageous if the risk of contaminating the injection needle is further reduced or minimized.

Some solutions of providing sealing between components of a needle assembly have been developed. For instance document WO 2010/094916 discloses a drug container and delivery mechanism having a needle hub with an injection needle provided with a proximal pointed end and a distal pointed end. A sealing element is arranged between the needle hub and a fastening component, intended to be connected to a medicament container. A further sealing element is arranged between the needle hub and a cap member.

When the assembly is to be used, the user manually removes the cap member, and then penetrates an injection site. It is only then that the distal end of the needle penetrates a covering membrane in order to obtain access to the medicament inside the container. This is a drawback in many instances and for many members that the passage for the medicament through the injection needle is created at the instance of the penetration. In many cases it is clearly desirable that a passage is created beforehand such that e.g. priming may be performed.

### BRIEF DESCRIPTION OF INVENTION

In the present application, when the term "distal part/end" is used, this refers to the part/end of the assembly, or the parts/ends of the members thereof, which is/are located the furthest away from the medicament delivery site of the patient. Correspondingly, when the term "proximal part/end" is used, this refers to the part/end of the assembly, or the parts/ends of the members thereof, which, is/are located closest to the medicament delivery site of the patient.

The aim of the present invention is to remedy the drawbacks of the state of the art injection needle assemblies. This aim is obtained by an injection needle assembly comprising the features of the independent claim 1. Preferable embodiments of the invention form the subject of the dependent patent claims.

The injection needle assembly according to the invention has opposite distal and proximal ends, and comprises a retainer member configured to be connected to a medicament container; a hub configured to be coaxially displaceable within the retainer member and provided with an injection needle having proximal and distal pointed ends; and a cap member interactively connected to the hub and to the retainer member; wherein the injection needle assembly further comprises a sealing member arranged between said cap member and said retainer member for preventing contamination of said injection needle before use. The sealing member comprises an elastic member, is generally ring-shaped and is attached to said retainer member. This is in order to increase the sealing properties of the sealing member, and maintain a sealing action even if there is some movement between the cap member and the retainer member. According to one aspect of the invention and in order to provide a proper sealing action of the sealing member, said sealing member is arranged with a proximally directed contact surface, and said cap member is arranged with a distally directed contact surface.

According to another aspect of the invention and in order to further ascertain the sealing action between the cap member and the retainer member, said sealing member is arranged such in relation to said retainer member and said cap member that said sealing member is compressed when said cap member is attached to said retainer member. Thus the sealing member provides enhanced protection of the injection needle in that the risk of any foreign matter passing between the cap member and the retainer member is greatly reduced. Thereby it is ensured that the sterility is kept intact until the cap member is removed from the retainer member.

According to a further aspect of the invention, the distal pointed end is arranged to penetrate a membrane arranged at the proximal end of the medicament container.

According to yet another aspect of the invention, the hub comprises on its outer circumferential surface a distal engagement member and a proximal engagement member.

According to another aspect of the invention, the retainer member comprises a distal tubular part having a container attachment member, a proximal part having an outer engagement member on its outer circumferential surface, and a central passage having opposite distal and proximal ends and an inner engagement member on its inner circumferential surface adapted to receive the distal engagement member of the hub.

According to a further aspect of the invention, the cap member comprises on its inner circumferential surface a first engagement member arranged to cooperate with the proximal engagement member of the hub, and a second engagement member arranged to cooperate with the outer engagement member of the retainer member.

According to yet another aspect of the invention, the first engagement member of the cap member is arranged to cooperate with the proximal engagement member of the hub, such that the hub is allowed to be axially displaceable but prevented to be rotated in relation to the cap member.

According to another aspect of the invention, wherein the second engagement member of the cap member is arranged to cooperate with the outer engagement member of the retainer member such that the cap member is allowed to be axially displaceable in relation to the retainer member from a first position, in which the cap member is prevented to be removed from the retainer member, to a second position, in which the cap member is allowed to be removed from the retainer member.

According to a further aspect of the invention, wherein the inner engagement member of the retainer member is arranged to cooperate with the distal engagement member of the hub such that the hub is allowed to be coaxially displaceable in relation to the retainer member from a disconnected position in which the distal engagement member is positioned at the proximal end of the central passage to a connected position in which the distal engagement member is positioned at the distal end of the central passage.

According to yet another aspect of the invention, the hub is displaced from the disconnected to the connected position when the cap member is displaced from the first to the second position.

According to another aspect of the invention, the inner engagement member of the retainer member and the distal engagement member of the hub are guiding members, and the second engagement member of the cap member and the outer engagement member of the retainer member are also guiding members.

According to yet another aspect of the invention, the inner engagement member of the retainer member and the distal engagement member of the hub are threaded members; and the second engagement member of the cap member and the outer engagement member of the retainer member are threaded members.

According to a further aspect of the invention, a medicament delivery device comprises an injection needle assembly according to any of the preceding aspects of the invention.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
- Fig. 1: is a perspective view of an injection needle assembly according to the present invention,
- Fig. 2: is an exploded view of the assembly of Fig. 1,
- Fig. 3: is a cross-sectional view of the assembly of Fig. 1,
- Figs. 4-5: are detailed views of the assembly of Fig. 1, and
- Fig. 6: is a perspective view of the assembly of Fig. 1 in an operational state.

### DETAILED DESCRIPTION OF THE INVENTION

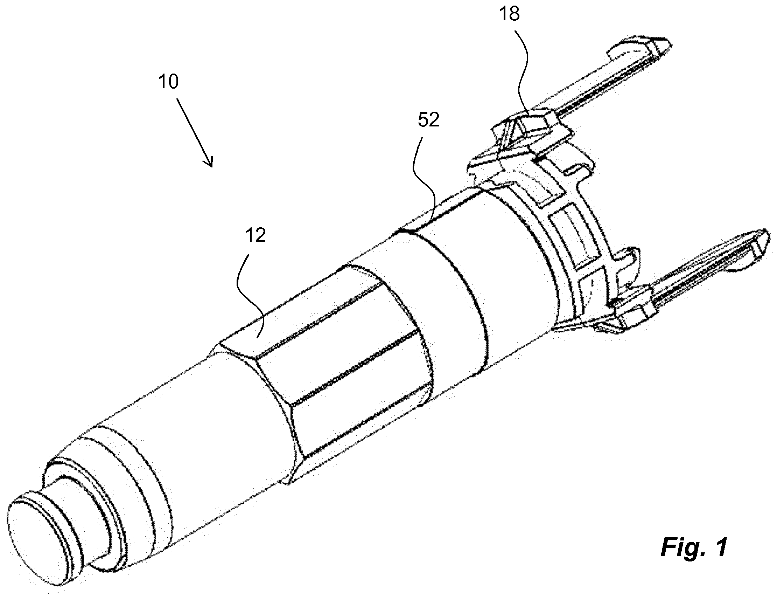

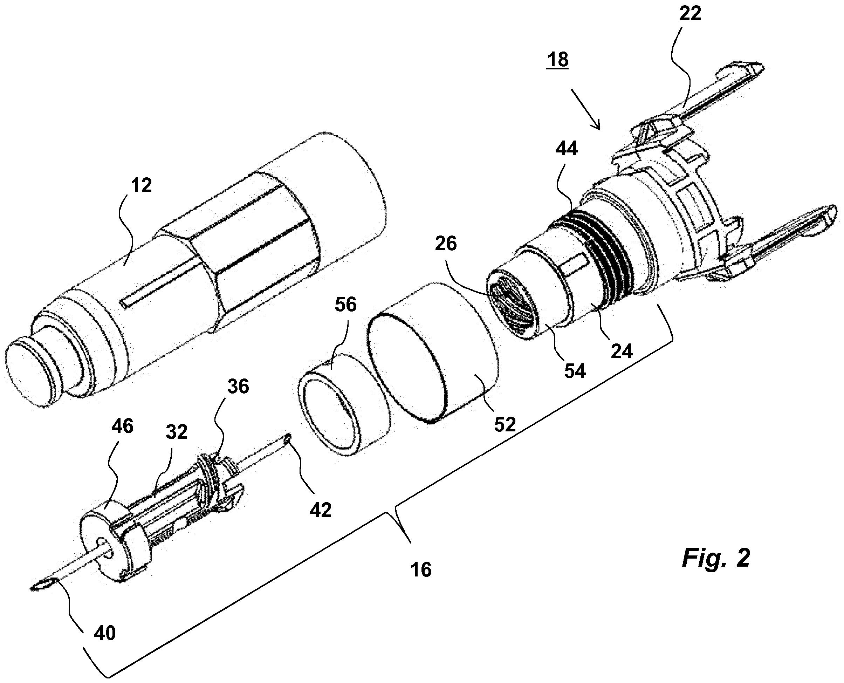

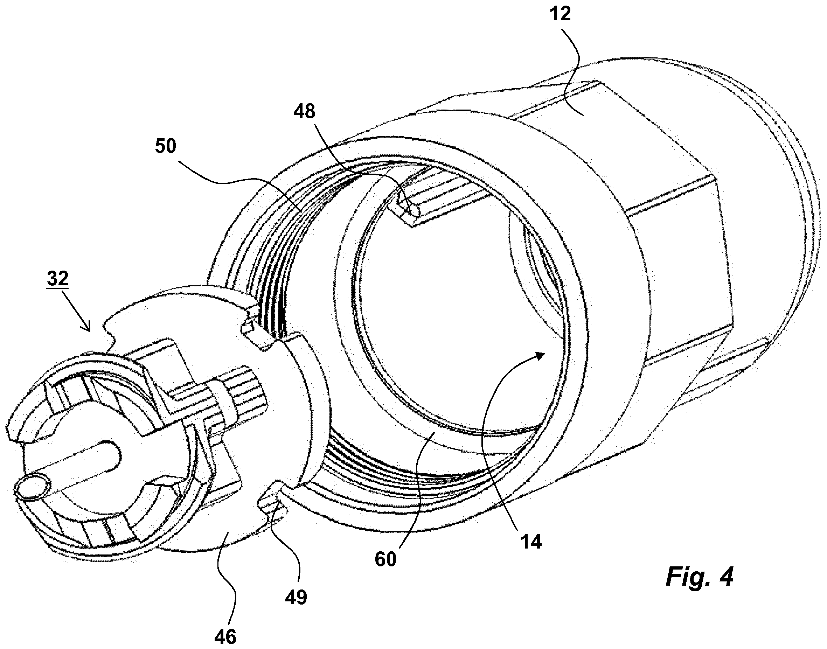

Figure 1 shows an injection needle assembly 10 according to the present invention. The injection needle assembly 10 has opposite distal and proximal ends. It comprises a retainer member 18 configured to be connected to a medicament container (not shown) in medicament delivery device; a hub 32 configured to be coaxially displaceable within the retainer member and provided with an injection needle 38 having proximal 40 and distal 42 pointed ends; and a cap member 12 interactively connected to both the hub and to the retainer member. The distal end of the cap member is arranged with an opening 14, Fig. 4, accessing the interior of the cap member 12, wherein an injection part 16 of the needle assembly is enclosed, Fig. 2. The distal pointed end 42 of the injection needle is arranged to penetrate a membrane arranged at the proximal end of the medicament container. According to the present invention, the injection needle assembly 10 further comprises a sealing member 56 arranged between said cap member 12 and said retainer member 18 for preventing contamination of said injection needle 38 before use.

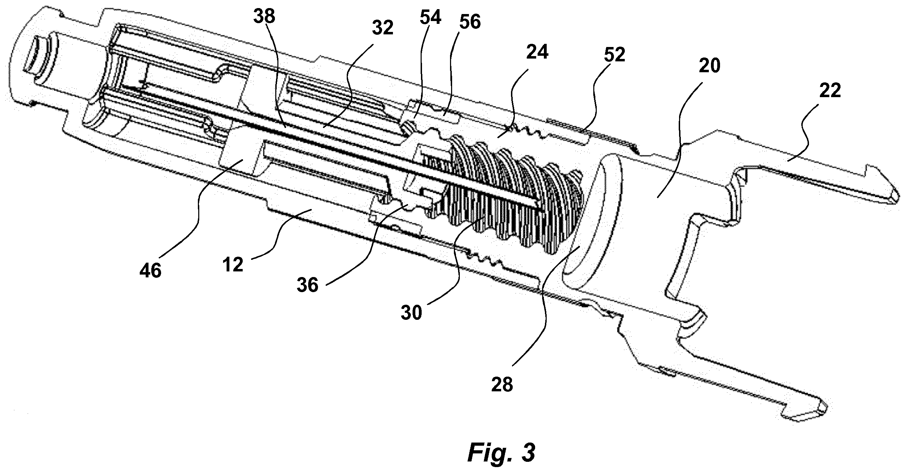

The retainer member 18 is arranged with a distal tubular part 20, Fig. 3, having a container attachment member into which a neck portion of a medicament container may fit, and where the medicament container is intended to be attached either by threads, by gluing or by welding, for example. The retainer member 18 is further provided with distally extending fastening means 22 for attachment to a housing part of a medicament delivery device.

The retainer member 18 is further arranged with a proximal tubular part 24 having an outer engagement member 44 on its outer circumferential surface, and a central passage 26. Said central passage has opposite distal and proximal ends and an inner engagement member 30 on its inner circumferential surface. A transversal extending wall 28, Fig. 3, is separating the distal tubular part 20 and the central passage 26, as will be described below. In the embodiment shown in Fig. 3, the inner engagement member 30 of the passage 26 is arranged with threads 30. In a further embodiment, (not shown), the inner engagement member 30 of the passage 26 is arranged with guiding members e.g. ribs or grooves.

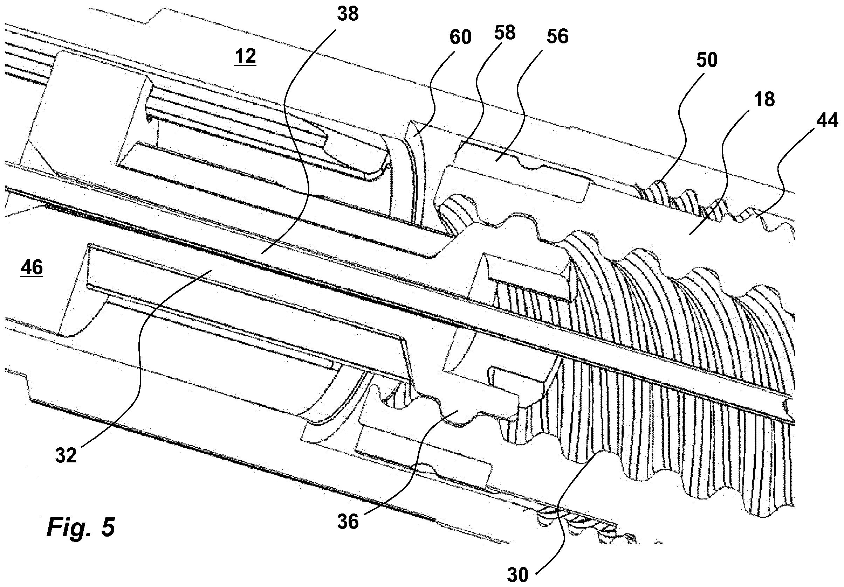

The hub 32 is a tubular shaped member, Figs. 2 and 5, comprising on its outer circumferential surface a distal engagement member 36 and a proximal engagement member 46. In the embodiment shown in Fig. 3, the distal engagement member 36 of the hub 32 is a threaded member designed to interact with the threads 30 of the central passage 26. In a further embodiment, (not shown), the distal engagement member 36 of the hub 32 is a guiding member e.g. ribs or grooves designed to interact with the corresponding guiding member in the passage 26. The injection needle 38 is attached through the hub 32, having proximal 40 and distal 42 pointed ends.

The cap member 12 is a tubular shaped member, Fig. 4, comprising on its inner circumferential surface a first engagement member 48 arranged to cooperate with the proximal engagement member 46 of the hub 32, and a second engagement member 50 arranged to cooperate with the outer engagement member 44 of the retainer member 18.

The proximal engagement member 46 of the injection hub 32, Fig. 4, is arranged with a number of longitudinally extending grooves or cut-outs 49 and the first engagement member 48 is a longitudinally extending rib. Each groove or cut-out 49 is intended to receive a longitudinally extending rib, such that a rotational lock is obtained between the cap member 12 and the hub 32, while allowing movement in the longitudinal direction between the two i.e. the hub 32 is allowed to be axially displaceable but prevented to be rotated in relation to the cap member 12.

The second engagement member 50 of the cap member 12 is arranged to cooperate with the outer engagement member 44 of the retainer member 18 such that the cap member 12 is allowed to be axially displaceable in relation to the retainer member from a first position, in which the cap member 12 is prevented to be removed from the retainer member, to a second position, in which the cap member 12 is allowed to be removed from the retainer member.

The inner engagement member 30 of the retainer member 18 is arranged to cooperate with the distal engagement member 36 of the hub 32 such that the hub 32 is allowed to be coaxially displaceable in relation to the retainer member 18 from a disconnected position in which the distal engagement member 36 is positioned at the proximal end of the central passage 26 to a connected position in which the distal engagement member 36 is positioned at the distal end of the central passage 26.

In the embodiment shown in Fig. 3, the second engagement member 50 of the cap member 12 and the outer engagement member 44 of the retainer member 18 are threaded members. The threads 44 are intended to cooperate with threads 50, Fig.4, for releasibly attaching the cap member 12 to the retainer member 18. In a further embodiment, (not shown), the second engagement member 50 of the cap member 12 and the outer engagement member 44 of the retainer member 18 are guiding members e.g. ribs or grooves.

A tamper indication member 52, Figs. 1 and 2, is arranged, comprising an adhesive label or sticker attached outside the interface between the cap member 12 and the retainer member 18.

Further, the retainer member 18 is arranged with a circumferentially recessed proximal portion 54, Fig. 2. This portion is arranged to accommodate the sealing member 56. In the embodiment shown, the sealing member is a ring-shaped member made of an elastic material, having a proximally facing circumferential surface 58. This surface 58 is intended to be in contact with a distally directed circumferential ledge 60, Figs. 4 and 5, on the inner surface of the cap member12. The sealing member 56, the recessed portion 54 and the circumferential ledge 60 are designed such that the sealing member 56 is compressed somewhat when the cap member12 is attached to the retainer member 18. The sealing member 56 thus creates an effective seal between the cap member12 and the retainer member 18, thereby preventing any contamination of the injection needle 44 before use of the needle assembly.

When the injection needle assembly is to be used, the cap member 12 is displaced from the first to the second position and because of the connection with the hub 32; the latter is also displaced from the disconnected to the connected position.

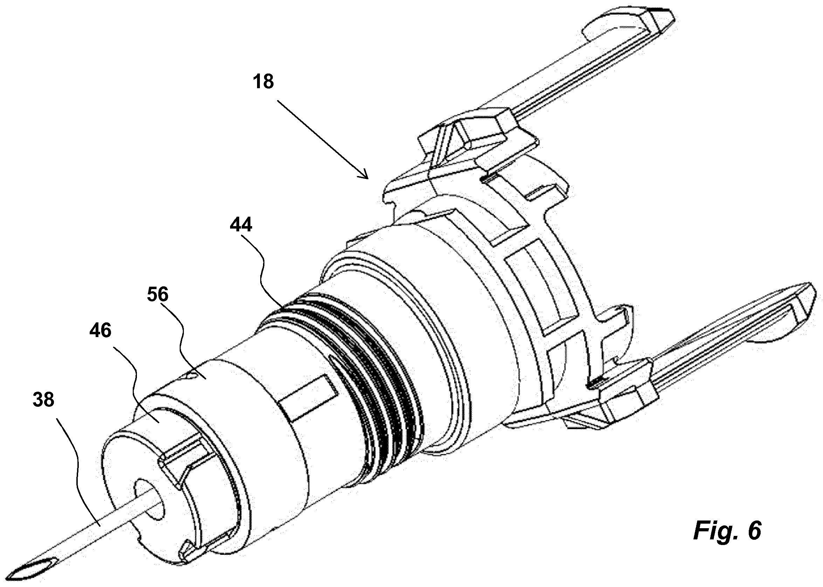

According to the embodiment shown in the figures, when the cap member12 is turned, the hub 32 is also turned. However, the threads between the cap member 12 and the retainer member 18 have a different direction of the pitch than the threads between the hub 32 and the retainer member 18. Therefore, when the cap member 12 is turned for removing it from the retainer member 18 in the proximal direction, the turning of the injection hub 32 causes it to move in the distal direction into the retainer member 18, Fig.6, whereby the pointed distal end 42 of the injection needle 38 penetrates the transversal wall 28 of the retainer member 18 as well as the membrane of the medicament container and due to the different direction of the pitch the cap member is screwed off the hub and may be removed, Fig. 6.

According to a further embodiment, when the cap member12 is first axially displaced towards the distal end of the assembly and the turned in relation to the retainer member 18, the hub 32 is also is axially displaced towards the distal end of the assembly and turned in relation to the retainer member. For removing the cap member, the guiding members between the cap member 12 and the retainer member 18 are configured such that the cap member can only be axially displaced towards the distal end of the assembly, turned and then displaced towards the proximal end of the assembly. The guiding members between the hub 32 the retainer member 18 are configured such that the hub is can be axially displaced towards the distal end of the assembly and turned. Therefore, when the cap member 12 is pushed, turned and pulled for removing it from the retainer member 18, the axial displacement of the injection hub 32 causes it to move in the distal direction into the retainer member 18, whereby the pointed distal end 42 of the injection needle 38 penetrates the transversal wall 28 of the retainer member 18 as well as the membrane of the medicament container.

It is to be understood that the embodiment described above and shown in the drawings is to be regarded only as a non-limiting example of the invention and that it may be modified in many ways within the scope of the patent claims.

## Claims

1. An injection needle assembly (10) having opposite distal and proximal ends, comprising:
- a retainer member (18) configured to be connectable to a medicament container;
- a hub (32) configured to be coaxially displaceable within the retainer member and provided with an injection needle (38) having proximal and distal pointed ends (40; 42); and
- a cap member (12) interactively connected to both the hub and to the retainer member;
**characterised in that** it further comprises a sealing member (56) arranged between said cap member (12) and said retainer member (18) for preventing contamination of said injection needle (38) before use, wherein said sealing member (56) comprises a generally ring-shaped elastic member.

2. The injection needle assembly according to claim 1, wherein said sealing member (56) is attached to said retainer member (18).

3. The injection needle assembly according to claim 2, wherein said sealing member (56) is arranged with a proximally directed contact surface (58), and wherein said cap member (12) is arranged with a distally directed contact surface (60).

4. The injection needle assembly according to claim 3, wherein said sealing member (56) is arranged such in relation to said retainer member (18) and said cap member (12) that said sealing member (56) is compressed when said cap member (12) is attached to said retainer member (18).

5. The injection needle assembly according to any one of the preceding claims 1-4, wherein the distal pointed end (42) is arranged to penetrate a membrane arranged at the proximal end of the medicament container.

6. The injection needle assembly according to any one of the preceding claims 1-5, wherein the hub (32) comprises on its outer circumferential surface a distal engagement member (36) and a proximal engagement member (46).

7. The injection needle assembly according to claim 6, wherein the retainer member (18) comprises a distal tubular part (20) having a container attachment member, a proximal tubular part (24) having an outer engagement member (44) on its outer circumferential surface, and a central passage (26) having opposite distal and proximal ends and an inner engagement member (30) on its inner circumferential surface adapted to receive the distal engagement member (36) of the hub (32).

8. The injection needle assembly according to claim 7, wherein the cap member (12) comprises on its inner circumferential surface a first engagement member (48) arranged to cooperate with the proximal engagement member (46) of the hub (32), and a second engagement member (50) arranged to cooperate with the outer engagement member (44) of the retainer member (18).

9. The injection needle assembly according to claim 8, wherein the first engagement member (48) of the cap member (12) is arranged to cooperate with the proximal engagement member (46) of the hub, such that the hub (32) is allowed to be axially displaceable but prevented to be rotated in relation to the cap member (12).

10. The injection needle assembly according to claim 9, wherein the second engagement member (50) of the cap member (12) is arranged to cooperate with the outer engagement member (44) of the retainer member (18) such that the cap member (12) is allowed to be axially displaceable in relation to the retainer member from a first position, in which the cap member (12) is prevented to be removed from the retainer member, to a second position, in which the cap member (12) is allowed to be removed from the retainer member.

11. The injection needle assembly according to claim 10, wherein the inner engagement member (30) of the retainer member (18) is arranged to cooperate with the distal engagement member (36) of the hub (32) such that the hub (32) is allowed to be coaxially displaceable in relation to the retainer member (18) from a disconnected position in which the distal engagement member (36) is positioned at the proximal end of the central passage (26) to a connected position in which the distal engagement member (36) is positioned at the distal end of the central passage (26).

12. The injection needle assembly according to claim 11, wherein the hub (18) is displaced from the disconnected to the connected position when the cap member (12) is displaced from the first to the second position.

13. The injection needle assembly according to claim 12, wherein the inner engagement member (30) of the retainer member (18) and the distal engagement member (36) of the hub (32) are guiding members.

14. The injection needle assembly according to claim 13, wherein the second engagement member (50) of the cap member (12) and the outer engagement member (44) of the retainer member (18) are guiding members.

15. The injection needle assembly according to claim 12, wherein the inner engagement member (30) of the retainer member (18) and the distal engagement member (36) of the hub (32) are threaded members.

16. The injection needle assembly according to claim 15, wherein the second engagement member (50) of the cap member (12) and the outer engagement member (44) of the retainer member (18) are threaded members.

17. Medicament delivery device comprising an injection needle assembly according to any one of the preceding claims.

## Patentansprüche

1. Injektionsnadeleinrichtung (10) aufweisend gegenüberliegende distale und proximale Enden, umfassend:
- ein Rückhalteelement (18), das konfiguriert ist, um mit einem Medikamentenbehälter verbindbar zu sein;
- eine Nabe (32), die konfiguriert ist, um innerhalb des Rückhalteelements koaxial verschiebbar zu sein und mit einer Injektionsnadel (38) aufweisend proximale und distale spitze Enden (40; 42) ausgestattet ist; und
- ein Kappenelement (12), das sowohl mit der Nabe als auch mit dem Rückhalteelement interaktiv verbunden ist;
**dadurch charakterisiert, dass** sie ferner ein Versiegelungselement (56) umfasst, das zwischen dem Kappenelement (12) und dem Rückhalteelement (18) angeordnet ist, um eine Verunreinigung der Injektionsnadel (38) vor dem Verwenden zu verhindern, wobei das Versiegelungselement (56) ein im Allgemeinen ringförmiges elastisches Element umfasst.

2. Injektionsnadeleinrichtung nach Anspruch 1, wobei das Versiegelungselement (56) am Rückhalteelement (18) angebracht ist.

3. Injektionsnadeleinrichtung nach Anspruch 2, wobei das Versiegelungselement (56) mit einer proximal zugewandten Kontaktoberfläche (58) angeordnet ist, und wobei das Kappenelement (12) mit einer distal zugewandten Kontaktoberfläche (60) angeordnet ist.

4. Injektionsnadeleinrichtung nach Anspruch 3, wobei das Versiegelungselement (56) im Verhältnis zum Rückhalteelement (18) und zum Kappenelement (12) derart angeordnet ist, dass das Versiegelungselement (56) komprimiert wird, wenn das Kappenelement (12) am Rückhalteelement (18) angebracht wird.

5. Injektionsnadeleinrichtung nach einem der vorhergehenden Ansprüche 1 bis 4, wobei das distale spitze Ende (42) angeordnet ist, um eine am proximalen Ende des Medikamentenbehälters angeordnetes Membran zu penetrieren.

6. Injektionsnadeleinrichtung nach einem der vorhergehenden Ansprüche 1 bis 5, wobei die Nabe (32) an ihrer äußeren Umfangsfläche ein distales Eingriffselement (36) und ein proximales Eingriffselement (46) umfasst.

7. Injektionsnadeleinrichtung nach Anspruch 6, wobei das Rückhalteelement (18) umfasst: ein distales rohrförmiges Teil (20) aufweisend ein Behälterbefestigungselement, ein proximales rohrförmiges Teil (24) aufweisend ein äußeres Eingriffselement (44) an seiner äußeren Umfangsfläche, und einen zentralen Durchgang (26) aufweisend gegenüberliegende distale und proximale Enden und ein inneres Eingriffselement (30) an seiner inneren Umfangsfläche, das angepasst ist, um das distale Eingriffselement (36) der Nabe (32) aufzunehmen.

8. Injektionsnadeleinrichtung nach Anspruch 7, wobei das Kappenelement (12) an seiner inneren Umfangsfläche ein erstes Eingriffselement (48), das angeordnet ist, um mit dem proximalen Eingriffselement (46) der Nabe (32) zusammenzuwirken, und ein zweites Eingriffselement (50) umfasst, das angeordnet ist, um mit dem äußeren Eingriffselement (44) des Rückhalteelements (18) zusammenzuwirken.

9. Injektionsnadeleinrichtung nach Anspruch 8, wobei das erste Eingriffselement (48) des Kappenelements (12) angeordnet ist, um mit dem proximalen Eingriffselement (46) der Nabe derart zusammenzuwirken, dass die Nabe (32) in die Lage versetzt wird, axial verschiebbar zu sein, aber daran gehindert wird, im Verhältnis zum Kappenelement (12) gedreht zu werden.

10. Injektionsnadeleinrichtung nach Anspruch 9, wobei das zweite Eingriffselement (50) des Kappenelements (12) angeordnet ist, um mit dem äußeren Eingriffselement (44) des Rückhalteelements (18) derart zusammenzuwirken, dass das Kappenelement (12) in die Lage versetzt wird, im Verhältnis zum Rückhalteelement von einer ersten Position, in der das Kappenelement (12) daran gehindert wird, vom Rückhalteelement entfernt zu werden, zu einer zweiten Position axial verschiebbar zu sein, in der das Kappenelement (12) in die Lage versetzt wird, vom Rückhalteelement entfernt zu werden.

11. Injektionsnadeleinrichtung nach Anspruch 10, wobei das innere Eingriffselement (30) des Rückhalteelements (18) angeordnet ist, um mit dem distalen Eingriffselement (36) der Nabe (32) derart zusammenzuwirken, dass die Nabe (32) in die Lage versetzt wird, im Verhältnis zum Rückhalteelement (18) von einer getrennten Position, in der das distale Eingriffselement (36) am proximalen Ende des zentralen Durchgangs (26) positioniert ist, zu einer verbundenen Position koaxial verschiebbar zu sein, in der das distale Eingriffselement (36) am distalen Ende des zentralen Durchgangs (26) positioniert ist.

12. Injektionsnadeleinrichtung nach Anspruch 11, wobei die Nabe (18) von der getrennten zur verbundenen Position verschoben wird, wenn das Kappenelement (12) von der ersten zur zweiten Position verschoben wird.

13. Injektionsnadeleinrichtung nach Anspruch 12, wobei das innere Eingriffselement (30) des Rückhalteelements (18) und das distale Eingriffselement (36) der Nabe (32) Führungselemente sind.

14. Injektionsnadeleinrichtung nach Anspruch 13, wobei das zweite Eingriffselement (50) des Kappenelements (12) und das äußere Eingriffselement (44) des Rückhalteelements (18) Führungselemente sind.

15. Injektionsnadeleinrichtung nach Anspruch 12, wobei das innere Eingriffselement (30) des Rückhalteelements (18) und das distale Eingriffselement (36) der Nabe (32) Gewindeelemente sind.

16. Injektionsnadeleinrichtung nach Anspruch 15, wobei das zweite Eingriffselement (50) des Kappenelements (12) und das äußere Eingriffselement (44) des Rückhalteelements (18) Gewindeelemente sind.

17. Medikamentenabgabevorrichtung umfassend eine Injektionsnadeleinrichtung nach einem der vorhergehenden Ansprüche.

## Revendications

1. Ensemble aiguille d'injection (10) comportant des extrémités distale et proximale opposées, comprenant :
- un élément de retenue (18), conçu de façon à pouvoir être raccordé à un récipient de médicament,
- un raccord (32) conçu de façon à pouvoir subir un déplacement coaxial à l'intérieur de l'élément de retenue et pourvu d'une aiguille d'injection (38) comportant des extrémités pointues proximale et distale (40 ; 42), et
- un élément formant capuchon (12), raccordé d'une manière interactive tant au raccord qu'à l'élément de retenue ;
**caractérisé en ce qu'**il comprend en outre un élément d'étanchéité (56) disposé entre ledit élément formant capuchon (12) et ledit élément de retenue (18) dans le but d'empêcher une contamination de ladite aiguille d'injection (38) avant utilisation, ledit élément d'étanchéité (56) comprenant un élément élastique généralement annulaire.

2. Ensemble aiguille d'injection selon la revendication 1, dans lequel ledit élément d'étanchéité (56) est fixé audit élément de retenue (18).

3. Ensemble aiguille d'injection selon la revendication 2, dans lequel ledit élément d'étanchéité (56) est doté d'une surface de contact (58) dirigée dans la direction proximale, et dans lequel ledit élément formant capuchon (12) est doté d'une surface de contact (60) dirigée dans la direction distale.

4. Ensemble aiguille d'injection selon la revendication 3, dans lequel ledit élément d'étanchéité (56) est disposé, par rapport audit élément de retenue (18) et audit élément formant capuchon (12), de telle sorte que ledit élément d'étanchéité (56) soit comprimé quand ledit élément formant capuchon (12) est fixé audit élément de retenue (18).

5. Ensemble aiguille d'injection selon l'une quelconque des revendications précédentes 1 à 4, dans lequel l'extrémité pointue distale (42) est disposée de façon à pénétrer dans une membrane disposée à l'extrémité proximale du récipient de médicament.

6. Ensemble aiguille d'injection selon l'une quelconque des revendications précédentes 1 à 5, dans lequel le raccord (32) comprend sur sa surface circonférentielle extérieure un élément d'accrochage distal (36) et un élément d'accrochage proximal (46).

7. Ensemble aiguille d'injection selon la revendication 6, dans lequel l'élément de retenue (18) comprend une partie tubulaire distale (20) comportant un élément de fixation de récipient, une partie tubulaire proximale (24) comportant un élément d'accrochage extérieur (44) sur sa surface circonférentielle extérieure, et un passage central (26) comportant des extrémités distale et proximale opposées et un élément d'accrochage intérieur (30) sur sa surface circonférentielle intérieure, conçu pour recevoir l'élément d'accrochage distal (36) du raccord (32).

8. Ensemble aiguille d'injection selon la revendication 7, dans lequel l'élément formant capuchon (12) comprend sur sa surface circonférentielle intérieure un premier élément d'accrochage (48) disposé de façon à coopérer avec l'élément d'accrochage proximal (46) du raccord (32), et un second élément d'accrochage (50) disposé de façon à coopérer avec l'élément d'accrochage extérieur (44) de l'élément de retenue (18).

9. Ensemble aiguille d'injection selon la revendication 8, dans lequel le premier élément d'accrochage (48) de l'élément formant capuchon (12) est disposé de façon à coopérer avec l'élément d'accrochage proximal (46) du raccord, de telle sorte que le raccord (32) puisse subir un déplacement axial mais ne puisse subir de rotation par rapport à l'élément formant capuchon (12).

10. Ensemble aiguille d'injection selon la revendication 9, dans lequel le second élément d'accrochage (50) de l'élément formant capuchon (12) est disposé de façon à coopérer avec l'élément d'accrochage extérieur (44) de l'élément de retenue (18) de telle sorte que l'élément formant capuchon (12) puisse subir un déplacement axial par rapport à l'élément de retenue à partir d'une première position, dans laquelle l'élément formant capuchon (12) ne peut pas être enlevé de l'élément de retenue , jusqu'à une seconde position dans laquelle l'élément formant capuchon (12) peut être enlevé de l'élément de retenue.

11. Ensemble aiguille d'injection selon la revendication 10, dans lequel l'élément d'accrochage intérieur (30) de l'élément de retenue (18) est disposé de façon à coopérer avec l'élément d'accrochage distal (36) du raccord (32) de telle sorte que le raccord (32) puisse subir un déplacement coaxial par rapport à l'élément de retenue (18) à partir d'une position désaccouplée, dans laquelle l'élément d'accrochage distal (36) est positionné au niveau de l'extrémité proximale du passage central (26), jusqu'à une position raccordée, dans laquelle l'élément d'accrochage distal (36) est disposé au niveau de l'extrémité distale du passage central (26).

12. Ensemble aiguille d'injection selon la revendication 11, dans lequel le raccord (18) est déplacé de la position désaccouplée à la position raccordée quand l'élément formant capuchon (12) est déplacé de la première à la seconde position.

13. Ensemble aiguille d'injection selon la revendication 12, dans lequel l'élément d'accrochage intérieur (30) de l'élément de retenue (18) et l'élément d'accrochage distal (36) du raccord (32) sont des éléments de guidage.

14. Ensemble aiguille d'injection selon la revendication 13, dans lequel le second élément d'accrochage (50) de l'élément formant capuchon (12) et l'élément d'accrochage extérieur (44) de l'élément de retenue (18) sont des éléments de guidage.

15. Ensemble aiguille d'injection selon la revendication 12, dans lequel l'élément d'accrochage intérieur (30) de l'élément de retenue (18) et l'élément d'accrochage distal (36) du raccord (32) sont des éléments filetés.

16. Ensemble aiguille d'injection selon la revendication 15, dans lequel le second élément d'accrochage (50) de l'élément formant capuchon (12) et l'élément d'accrochage extérieur (44) de l'élément de retenue (18) sont des éléments filetés.

17. Dispositif d'administration de médicament, comprenant un ensemble aiguille d'injection selon l'une quelconque des revendications précédentes.
